# EUROPÄISCHE PATENTSCHRIFT

(11) **EP 1 533 614 B1**
(45) Veröffentlichungstag und Bekanntmachung des Hinweises auf die Patenterteilung: **21.09.2011**
(21) Anmeldenummer: 04026357.6
(22) Anmeldetag: 05.11.2004
(51) Int. Cl.: G01N 33/487, G01N 27/30, G01N 27/333

(54) **Sensorkarte zur Bestimmung von Analyten in Flüssigkeits- oder Gasproben und Verfahren zur Herstellung einer solchen Sensorkarte**
Sensor card for determining analytes in liquid and gaseous samples and method for producing same
Carte test pour la détermination de substances dans un échantillon liquide ou gazeux et sa procédé de production

(30) Priorität: 18.11.2003 DE 10353938
(43) Veröffentlichungstag der Anmeldung: 25.05.2005
(73) Patentinhaber: Fresenius Medical Care Deutschland GmbH, 61352 Bad Homburg v.d.H. (DE)
(72) Erfinder: Abel, Petra, 61169 Friedberg (DE); Schrörs, Alexander, 60318 Frankfurt (DE); Chemnitius, Gabriele, Dr., 61352 Bad Homburg (DE); Mager, Gerhard, Dr., 61352 Bad Homburg-Obererlenbach (DE); Häcker, Jürgen, 61267 Neu-Anspach (DE)
(74) Vertreter: Luderschmidt, Schüler & Partner

(56) Entgegenhaltungen:
- WO-A-02/097415
- DE-A1- 19 631 530
- DE-A1- 19 747 875
- US-A- 5 405 510
- HAMPP N ET AL: "DESIGN AND APPLICATION OF THICK-FILM MULTISENSORS" SENSORS AND ACTUATORS A, ELSEVIER SEQUOIA S.A., LAUSANNE, CH, Bd. A31, Nr. 1 / 3, 1. März 1992 (1992-03-01), Seiten 144-148, XP000276418 ISSN: 0924-4247
- EGGENSTEIN C ET AL: "A DISPOSABLE BIOSENSOR FOR UREA DETERMINATION IN BLOOD BASED ON AN AMMONIUM-SENSITIVE TRANSDUCER" BIOSENSORS & BIOELECTRONICS, ELSEVIER SCIENCE PUBLISHERS, BARKING, GB, Bd. 14, Nr. 1, 1. Januar 1999 (1999-01-01), Seiten 33-41, XP001020406 ISSN: 0956-5663

## Beschreibung

Die vorliegende Erfindung betrifft eine Sensorkarte zur Bestimmung von Analyten in Flüssigkeits- oder Gasproben mit flächig miteinander verbundenen Folien. Ferner betrifft die Erfindung ein Verfahren zur Herstellung einer solchen Sensorkarte.

Derartige Sensorkarten kommen in Meßinstrumenten bzw. Analysensystemen zum Einsatz, bei denen eine Flüssigkeits- oder Gasprobe einer chemischen Analyse unterzogen wird, wobei die quantitative Bestimmung von verschiedenen Analyten in gasförmigen und flüssigen Proben möglich ist. Derartige Analysensysteme werden verstärkt in der Medizintechnik, der Prozeßkontrolle sowie der Lebensmittel- und Umweltanalytik eingesetzt.

Die Analyse von Parametern von Körperflüssigkeiten im Rahmen des point-of-care (POC) Testings im Bereich der medizinischen Diagnostik stellt hierbei ein Haupteinsatzgebiet dieser Analysensysteme dar. Bei der Behandlung von akut und schwer erkrankten Patienten ist es für die behandelnden Ärzte wichtig, schnell und einfach präzise Informationen über beispielsweise die physiologischen Blutparameter der Patienten zu erhalten. Zu diesem Blutparametern gehören die Blutgase (pO₂, pCO₂, pH), die Elektrolyten (Na, K, Ca, Cl), die Leitfähigkeit des Blutes und daraus abgeleitet der Hämatokrit und die Metaboliten (Glucose, Lactat, Harnstoff, Creatinin). Zunehmend wird dabei der Ort der Analyse vom Zentrallabor in die Nähe des Patienten verlagert. Die Analysen müssen daher nicht mehr von spezialisiertem Laborpersonal durchgeführt werden, sondern können von dem Personal, das auch den Patienten betreut, durchgeführt werden. Dadurch wird ein beträchtlicher Zeitgewinn erzielt, der einer schnelleren und effizienteren Behandlung des Patienten zugute kommt.

Bei der Entwicklung der dazu eingesetzten Analysensysteme ist es insofern besonders wichtig, dass die Geräte einfach und sicher zu bedienen sind, keine komplizierten und zeitaufwendigen Wartungsmaßnahmen benötigen und die Analysenkosten pro Probe so gering wie möglich gehalten werden. Es gibt bereits verschiedene klinische Analysensysteme, die für diese Aufgabe eingesetzt werden. Sie verwenden überwiegend elektrochemische Sensoren zur Messung der Analyte. In einigen Systemen werden dabei Sensoren konventioneller Bauform bestehend aus Elektrodenkörper, Innenelektrolyt und analytselektiver Membran verwendet, die lange halten, dafür aber immer wieder zeitaufwendig gewartet werden müssen.

Neuere Analysengeräte verwenden zunehmend wartungsfreie, planare Sensoren, die u.a. mit Verfahren der Dünn- und Dickschichttechnik in großen Serien hergestellt werden können. Mehrere Sensoren, von denen jeder einen Analyt spezifisch bestimmen kann, werden auf einem Substrat aufgebracht. In der Durchflußzelle werden zur Ermittlung der Analytkonzentrationen Kalibrierlösung und Flüssigkeitsprobe über die Sensoren gegeben.

So wurde im Rahmen des 17. Internationalen Symposiums der *International Federation of Clinical Chemistry and Laboratory Medicine* (IFCC) vom 4. bis 7. Juni 1998 in Nizza, Frankreich, eine neue Generation von Sensorkarten vorgestellt, die die sogenannte Doppel-Matrix-Membran (DMM) Technologie nutzen. Die bekannten Sensorkarten mit Doppel-Matrix-Membran weisen eine Sensorfolie mit Öffnungen auf, die der zu untersuchenden Flüssigkeitsprobe in Gebrauchsstellung zugewandt wird. Auf der der Flüssigkeitsprobe abgewandten Seite der Folie ist eine Deckfolie angeordnet, auf der elektrische Leiterbahnen aus Silber/Silberchlorid aufgedruckt sind, wobei diese Leiterbahnen der Sensorfolie zugewandt sind. Zwischen der Sensorfolie und der Deckfolie sind Trägermaterialien für ionenselektive DMM-Sensoren angeordnet, die einerseits an eine Öffnung in der Sensorfolie und andererseits an eine der Leiterbahnen auf der Deckfolie angrenzen. In dieser Anordnung werden die Folien und die Sensoren verbunden, wobei die Öffnungen in der Sensorfolie kleiner als die Sensorfläche ausgebildet sind, so dass der Rand der Öffnung den Rand des Sensors niederdrückt, was letztlich zu einer Erhöhung des Randes der Öffnung fuhrt, Der elektrische Abgriff erfolgt unmittelbar an den Leiterbahnen, die sich zu diesem Zweck bis zum Rand der Deckfolie erstrecken, die größer als die Sensorfolie ausgebildet ist, so dass ein Teil der Leiterbahn nicht durch die Sensorfolie verdeckt und abgreifbar ist.

Innerhalb des Analysensystems ist die bekannte DMM-Sensorkarte mit einer Platte flächig verbunden, in der sich eine oder mehrere Nuten befinden. Durch diese Verbindung entsteht ein Strömungskanal für die Flüssigkeitsprobe, der von der Wandung der Nuten einerseits und von der Sensorfolie der DMM-Sensorkarte andererseits begrenzt ist. Die Öffnungen in der Sensorfolie weisen dabei zu dem Strömungskanal, so dass die Sensoren mit der durch den Strömungskanal fließenden Flüssigkeitsprobe benetzt werden können. Die bekannte DMM-Sensoxkarte hat den Nachteil, dass diese keine sichere Abdichtung der Strömungskanäle innerhalb der Platte gewährleistet.

Die DE 197 47 875 A1 beschreibt eine Sensorkarte, die über eine Sensorfolie, eine Deckfolie und eine Zwischenfolie verfügt. In einer Ausnehmung der Zwischenteile befindet sich ein Sensor. Der elektrische Abgriff erfolgt über Kontakte und Kontaktbahnen, die zu Kontaktfahnen führten. Die Kontakte, Kontaktbahnen und -fahnen befinden sich auf der Seite der Deckfolie, die der Zwischenfolie zugewandt ist. Der Sensor kommt mit der Probe über eine Öffnung in der Sensorfolie in Kontakt. Bei der bekannten Sensorkarte ist die Kontaktierung des Sensors insofern nachteilig, als Kontaktbahnen und Kontaktfahnen auf der Seite der Deckfolie aufgebracht sind, die der Zwischenfolie zugewandt ist. Dadurch entsteht das Problem, dass sich die Kontaktfahnen nicht ohne weiteres abgreifen lassen. Das Problem wird dadurch gelöst, dass die Deckfolie einerseits und die Zwischen- und Sensorfolie andererseits so übereinander angeordnet werden, dass der Bereich der Deckfolie, an dem die Kontaktfahnen angeordnet sind, nach außen vorsteht.

Die WO 02/097415 A2 beschreibt einen Sauerstoff-Sensor, der über eine Arbeits-Elektrode, eine Referenz-Elektrode und eine Masse-Elektrode verfügt. Bei einer bevorzugten Ausführungsform des Sensors weist die Arbeits-Elektrode einen Platindraht auf, der im Zentrum einer Glasscheibe sitzt. An der einen Seite der Glasscheibe ist der Platindraht mit zwei Membranen abgedeckt, während der Draht auf der anderen Seite der Scheibe mit einer Elektrode kontaktiert ist. Der Platindraht ist auf der Seite, die der Kontaktierung mit der Elektrode abgewandt ist, einem Gasstrom ausgesetzt, während der elektrische Abgriff auf der Seite der Kontaktierung mit der Elektrode erfolgt.

Der vorliegenden Erfindung liegt somit die Aufgabe zu Grund, eine Sensorkarte zur Bestimmung von Analyten in Flüssigkeitsproben zu schaffen, die eine sichere Abdichtung der Strömungskanäle für die Flüssigkeitsprobe gewährleistet, sowie ein Verfahren zur Herstellung einer solchen Sensorkarte anzugeben.

Die Lösung dieser Aufgabe erfolgt an Hand der in den Patentansprüchen 1 bzw. 16 angegebenen Merkmale. Vorteilhafte Ausführungsformen der Erfindung sind Gegenstand der Unteransprüche.

Die erfindungsgemäße Sensorkarte zur Bestimmung von Analyten in Flüssigkeits- oder Gasproben weist flächig miteinander verbundene Folien auf, Die der Flüssigkeitsprobe zugewandte Folie wird als Sensorfolie bezeichnet und ist mit Öffnungen versehen. Der Flüssigkeitsprobe zugewandt bedeutet in diesem Zusammenhang, dass die Sensorfolie mit der Flüsstgkeitsprobe innerhalb der Strömungskanäle in Kontakt kommt, d.h. die Sensorkarte soll später, wie bereits unter Bezugnahme auf den Stand der Technik beschrieben, flächig mit der Platte verbunden werden, in der sich die Nuten befinden. Die Sensorkarte weist ferner eine Deckfolie auf, die der Probe abgewandt ist, d.h. die Deckfolie tritt nicht direkt mit der Probe in Kontakt. In der Deckfolie sind ferner Öffnungen zum elektrischen Abgriff vorgesehen. Ferner weist die Sensorkarte mindestens einen Sensor auf, der zwischen der Sensorfolie und der Deckfolie angeordnet ist. Als Sensoren kommen beispielsweise potentiometrische, amperometrische oder konduktometrische analytspezifische Sensoren in Frage. Erfindungsgemäß ist zwischen der Sensorfolie und der Deckfolie mindestens eine Zwischenfolie vorgesehen. In der Zwischenfolie sind Aussparungen vorgesehen sind, wobei der Sensor in einer der Aussparungen angeordnet ist.

Die erfindungsgemäße Sensorkarte hat den Vorteil, dass sie eine homogene Dicke aufweist und eine wellige Form vermieden wird. Dies ist darauf zurückzuführen, dass die Erhöhung, die durch das Anordnen der Sensoren zwischen Sensor- und Deckfolie zwangsläufig entsteht, durch die Zwischenfolie, in deren Aussparungen die Sensoren einliegen, kompensiert wird. Eine derart eben ausgebildete Sensorkarte kann besonders einfach und sicher flächig an der Platte mit Nuten befestigt werden, mit der die Sensorkarte die Strömungskanäle ausbildet, so dass eine hohe Dichtigkeit der Strömungskanäle gewährleistet ist. Darüber hinaus bietet die erfindungsgemäße Sensorkarte die Vorteile, dass diese sehr dünn ausgebildet ist, sehr viele Sensoren fassen kann und kostengünstig herstellbar ist.

In einer besonders vorteilhaften Ausführungsform der erfindungsgemäßen Sensorkarte ist die Dicke der Zwischenfolie derart gewählt, dass diese der Dicke des Sensors entspricht oder größer ist. Im ersten Fall wird eine absolut ebene Sensorkarte geschaffen, während im zweiten Fall kleinere nach innen weisende Wölbungen entstehen können, wobei dies im Hinblick auf die Dichtigkeit der Strömungskanäle noch immer vorteilhafter ist, als vorspringende Wölbungen, wie sie bei der bekannten DMM-Sensorkarte auftreten.

Um ein möglichst einfaches und sicheres elektrisches Abgreifen durch die Öffnungen in der Deckfolie zu ermöglichen, sind die Öffnungen in einer weiteren bevorzugten Ausführungsform der Erfindung mit einer ausgehärteten, elektrisch leitenden Paste gefüllt. Hierbei kann es sich vorzugsweise um Kohlepaste handeln.

In einer bevorzugten Ausführungsform der erfindungsgemäßen Sensorkarte ist der Sensor als ionenselektiver Sensor ausgebildet. Der ionenselektive Sensor kann einerseits über eine der Öffnungen in der Deckfolie elektrisch abgegriffen und andererseits über eine der Öffnungen in der Sensorfolie mit der Flüssigkeitsprobe benetzt werden.

Vorteilhafterweise ist die Öffnung zum elektrischen Abgriff des ionenselektiven Sensors in einer weiteren Ausführungsform der Erfindung versetzt zu der Öffnung in der Sensorfolie angeordnet. Unter versetzt ist hierbei zu verstehen, dass die Öffnung in der Deckfolie in der Draufsicht auf die Sensorkarte und die Öffnung in der Sensorfolie im Abstand zueinander angeordnet sind, wobei vorzugsweise keine Überschneidung der beiden Öffnungen besteht.

In einer weiteren Ausführungsform der erfindungsgemäßen Sensorkarte weist der ionenselektive Sensor ein in der Aussparung einliegendes Trägermaterial auf. Als Trägermaterial kommt beispielsweise saugfähiges Papier in Frage. Im Bereich der Öffnung der Sensorfolie ist das Trägermaterial mit einer ionenselektiven Reagenzmischung aktiviert bzw. funktionalisiert. Die Reagenzmischung füllt dabei die Öffnung in der Sensorfolie vollständig aus, so dass der ionenselektive Sensor gegenüber der Sensorfolie sicher abgedichtet ist.

Das Trägermaterial des ionenselektiven Sensors weist in einer besonders bevorzugten Ausführungsform an seiner der Öffnung zum elektrischen Abgriff zugewandten Seite eine leitfähige Beschichtung auf. Die leitfähige Beschichtung dient dem elektrischen Anschluß des ionsenselektiven Sensors und gewährleistet ein sicheres elektrisches Abgreifen durch die Öffnung in der Deckfolie.

In einer besonders bevorzugten Ausführungsform der Erfindung weist die Sensorfolie der Zwischenfolie zugewandte, aufgedruckte elektrische Leiterbahnen auf. Derartige Leiterbahnen können beispielsweise mittels Siebdruck aufgebracht werden und ebenfalls aus Kohlepaste bestehen. Durch den Siebdruck können auch besonders flache Leiterbahnen erzeugt werden, die ebenfalls zur Lösung der erfindungsgemäßen Aufgabe beitragen. Die Leiterbahnen können jeweils über die Öffnungen zum elektrischen Abgriff und mindestens eine der Aussparungen in der Zwischenfolie elektrisch angeschlossen werden. Man kann bei dieser Ausführungsform von einer Durchkontaktierung sprechen, die sich einfacher und platzsparender als das seitliche Abgreifen an der bekannten DMM-Sensorkarte gestaltet.

Der Sensor ist in einer weiteren Ausführungsform der erfindungsgemäßen Sensorkarte als Gassensor ausgebildet. Der Gassensor steht mit einer ersten Leiterbahn und einer zweiten Leiterbahn auf der Sensorfolie in Kontakt. Des weiteren kann der Gassensor über eine der Öffnungen in der Sensorfolie mit der Probe in Kontakt kommen.

Der Gassensor ist in einer weiteren Ausführungsform der erfindungsgemäßen Sensorkarte ein Kohlendioxidsensor, der aus einer Trägerfolie besteht, die eine der Sensorfolie zugewandte Beschichtung, vorzugsweise eine Bedruckung aus Silber/Silberchlorid- und einem pH-sensitiven-Material aufweist.

Der Gassensor ist in einer weiteren Ausführungsform der Erfindung ein Sauerstoffsensor. Der Sauerstoffsensor besteht aus einer Trägerfolie mit hierauf aufgebrachten, vorzugsweise aufgedruckten Schichten eines als Arbeitselektrode und eines als Referenzelektrode geeigneten Materials, die der Sensorfolie zugewandt sind.

Gemäß einer weiteren vorteilhaften Ausführungsform der Erfindung ist der Sensor ein Leitfähigkeitssensor. Dieser weist zwei elektrisch leitfähige, voneinander beabstandete Leitungsabschnitte auf, die auf der der Sensorfolie zugewandten Seite der Zwischenfolie aufgedruckt sind. Die Leitungsabschnitte können beispielsweise wie die Leiterbahnen mittels Siebdruck aufgebracht werden und aus einer getrockneten Kohlepaste bestehen. Der eine Leitungsabschnitt steht mit einer ersten Leiterbahn auf der Sensorfolie und der andere Leitungsabschnitt steht mit einer zweiten Leiterbahn auf der Sensorfolie in Kontakt. Die Leitfähigkeitssensoren können über Öffnungen in der Sensorfolie mit der Flüssigkeitsprobe benetzt und dadurch elektrisch leitend verbunden werden, um die Leitfähigkeit der Flüssigkeitsprobe zu ermitteln.

Um eine ausreichende Belüftung zu gewährleisten, ist bei einer vorteilhaften Ausführungsform der Sensorkarte in mindestens einer der Aussparungen in der Zwischenfolie eine Belüftungsmembran angeordnet. Die Belüftungsmembran grenzt einerseits an eine Belüftungsöffnung in der Sensorfolie und andererseits an eine Belüftungsöffnung in der Deckfolie an.

Zuweilen ist es bei den Analysensystemen wünschenswert, zwei voneinander getrennte Strömungskanäle innerhalb der Platte, auf der die Sensorfolie befestigt wird, durch einen schmalen Kanal zu verbinden, der nur durch größeren Aufwand in der Platte selbst erzeugt werden könnte. Um einen solchen Kanal zu erzeugen, ist in einer besonders bevorzugten Ausführungsform der erfindungsgemäßen Sensorkarte eine der Aussparungen in der Zwischenfolie länglich ausgebildet, wobei die längliche Aussparung mit mindestens zwei separaten Öffnungen in der Sensorfolie in Strömungsverbindung steht. Somit kann durch eine der Öffnungen in der Sensorfolie eine Flüssigkeit oder ein Gas, wie beispielsweise eine Reagenzlösung, in die längliche Aussparung gelangen, um von dort durch die zweite der beiden Öffnungen in der Sensorfolie wieder auszutreten. Dies kann beispielsweise dazu benutzt werden, einen Strömungskanal für eine Reagenzlösung mit einem Strömungskanal für eine Flüssigkeitsprobe zu verbinden. Eine Verbindung der Strömungskanäle für Reagenzlösung und Flüssigkeitsprobe ist beispielsweise dann vorzusehen, wenn der Innenelektrolyt einer Referenzelektrode, der in einem Beutel zur Verfügung gestellt werden kann, während der Messungen intermittierend über den Strömungskanal für die Reagenzlösung dem Probenkanal zugeführt werden soll.

In einer besonders bevorzugten Ausführungsform der erfindungsgemäßen Sensorkarte sind die beiden separaten Öffnungen in der Sensorfolie ebenfalls länglich ausgebildet und verlaufen quer, vorzugsweise im rechten Winkel, zu der länglichen Aussparung in der Zwischenfolie. Dies gewährleistet, dass die beiden länglichen Öffnungen in der Sensorfolie und die längliche Aussparung in der Zwischenfolie auch tatsächlich wie gewünscht in Strömungsverbindung stehen. Während bei punktförmigen Öffnungen in der Sensorfolie geringe Toleranzen beim Übereinanderlegen der Folien eingehalten werden müssten, um eine Strömungsverbindung zu gewährleisten, fallen bei länglichen Öffnungen in der Sensorfolie auch größere Verschiebungen der Folien zueinander nicht ins Gewicht, da die längliche Öffnung die längliche Aussparung in mehreren Punkten überlagern kann. Die Fertigung der Sensorkarte ist somit vereinfacht.

Das erfindungsgemäße Verfahren zur Herstellung einer Sensorkarte zur Bestimmung von Analyten in Flüssigkeits- oder Gasproben mit flächig miteinander verbundenen Folien weist folgende Verfahrensschritte auf. Zunächst wird eine Sensorfolie mit Öffnungen, eine Deckfolie mit Öffnungen zum elektrischen Abgriff und mindestens eine Zwischenfolie, in der Aussparungen vorgesehen sind, zur Verfügung gestellt. Danach wird mindestens ein Sensor oder der Teil eines Sensors in einer der Aussparungen der Zwischenfolie angeordnet. Abschließend werden die übereinander angeordneten Folien flächig miteinander verbunden, wobei die Zwischenfolie zwischen Sensorfolie und Deckfolie angeordnet ist.

Das erfindungsgemäße Verfahren sieht zwei Alternativen vor. Entweder kann der fertige Sensor oder nur ein Teil eines in einem nachfolgenden Verfahrensschritt noch fertigzustellenden Sensors in der Aussparung der Zwischenfolie angeordnet werden. So kann anstelle des fertigen Sensors auch nur das Trägermaterial des Sensors, das bereits mit Elektroden versehen sein kann, in die Zwischenfolie eingelegt werden. Nach dem Verbinden der Folien wird das Trägermaterial dann aktiviert bzw. funktionalisiert. Das Trägermaterial kann aus unterschiedlichen Materialien bestehen. Die Aktivierung bzw. Funktionalisierung kann dadurch erfolgen, dass auf das Trägermaterial durch die Öffnung in der Sensorfolie ein oder mehrere weitere Materialien aufgebracht werden, die der Aktivierung oder Funktionalisierung dienen. Der Einbau eines fertigen Sensors hat den Vorteil der einfachen Herstellung, während der Einbau eines noch zu aktivierenden bzw. funktionalisierenden Teils eines Sensors, den Vorteil hat, dass der Teil des Sensors mit dem aufzubringenden Material für die Aktivierung bzw. Funktionalisierung den fertigen Sensor gegenüber der Sensorfolie sicher abdichtet.

In einer besonders bevorzugten Ausführungsform des erfindungsgemäßen Verfahrens wird zumindest eine der Folien, d.h. Sensor- oder Deckfolie, vor dem Anordnen des Sensors oder Teils des Sensors in einer der Aussparungen mit der Zwischenfolie teilweise flächig verbunden. Ein solches Verfahren ermöglicht ein einfacheres Anordnen der Sensoren innerhalb der Aussparung in der Zwischenfolie, da durch die zum Teil befestigte Deck- oder Sensorfolie ein Herausfallen des Sensors verhindert wird.

In einer weiteren besonders vorteilhaften Ausführungsform wird sowohl die Sensorfolie als auch die Deckfolie vor dem Anordnen des Sensors oder Teils des Sensors in einer der Aussparungen mit der Zwischenfolie teilweise flächig verbunden. Neben dem in der vorbeschriebenen Ausführungsform genannten Vorteil, hat dies zusätzlich den Vorteil, dass bereits erkannt werden kann, ob die Öffnungen und Aussparungen in den Folien schon vor dem kompletten Verbinden richtig zueinander angeordnet sind.

Eine weitere Ausführungsform des erfindungsgemäßen Verfahrens ist dadurch gekennzeichnet, dass vor dem Zurverfügungstellen der Sensorfolie Leiterbahnen auf die der Zwischenfolie zuzuwendende Seite der Sensorfolie aufgedruckt werden, vorzugsweise durch Siebdruck. Siebdruck ist ein zuverlässiges Verfahren, das die Erzeugung besonders flacher Leiterbahnen ermöglicht.

In einer weiteren Ausführungsform der Erfindung werden vor dem Zurverfügungstellen der Zwischenfolie Leitungsabschnitte auf die der Sensorfolie zuzuwendende Seite der Zwischenfolie aufgedruckt, vorzugsweise durch Siebdruck. Siebdruck wird von der Fertigung auch auf der der Zwischenfolie zugewandten Seite der Deckfolie angestrebt. Im Prinzip kann auf beide Seiten der Zwischenfolie und die Innenseiten der Deck- und Sensorfolie gedruckt werden. Das Verbinden der Folien erfolgt vorzugsweise durch Kleben, Heißlaminieren, Hitzeverschweißen oder Hochfrequenzschweißen.

Einzelne oder alle Folien der Karte können lokal ausgespart sein, um zusammen mit der Umgebung der Sensorkarte weitere Funktionen zu ermöglichen und um eine exakte Positionierung der erfindungsgemäßen Sensorkarte gegenüber einer Platte mit Nut zu ermöglichen.

Die Sensorkarte kann so positioniert werden, dass, wenn notwendig, einzelne Öffnungen der Sensorfolie befüllt werden können, um die Sensoren zu aktivieren bzw. funktionalisieren. Für ionenselektiven Sensoren können die entsprechenden ionenselektiven Reagenzmischungen (Cocktails) aufgebracht werden. Die jeweiligen ionenselektiven Reagenzmischungen (Cocktails) für die verschiedenen ionenselektiven Sensoren werden in die betreffenden Öffnungen auf das Trägermaterial getropft und getrocknet. Auch können gaspermeable Membranen für Gassensoren aufdosiert und getrocknet werden. Es ist auch denkbar, die funktionalen Schichten vor dem endgültigen Zusammenfügen der Folien direkt auf die Sensorinlays aufzubringen. Andere bzw. zusätzliche Materialien für die Aktivierung bzw. Funktionalisierung, wie sie z.B. für Biosensoren eingesetzt werden, können analog aufgebracht werden.

Die Sensorkarte kann einfach an die analytischen Aufgaben angepasst werden, indem die Art und die Anzahl der in ihrer Zwischenfolie integrierten Sensoren auf die zu analysierenden Stoffe abgestimmt wird. Der Aufbau der Sensorkarte ist in dieser Hinsicht als modular anzusehen. Außerdem ist es auch möglich, weitere Sensoren direkt auf die Zwischenfolie zu drucken anstatt sie als Sensorinlays in die Aussparungen der Zwischenteile einzulegen.

Im Folgenden wird die Erfindung an Hand einer beispielhaften Ausführungsform unter Bezugnahme auf die beigefügten Figuren eingehender erläutert.

Es zeigen:

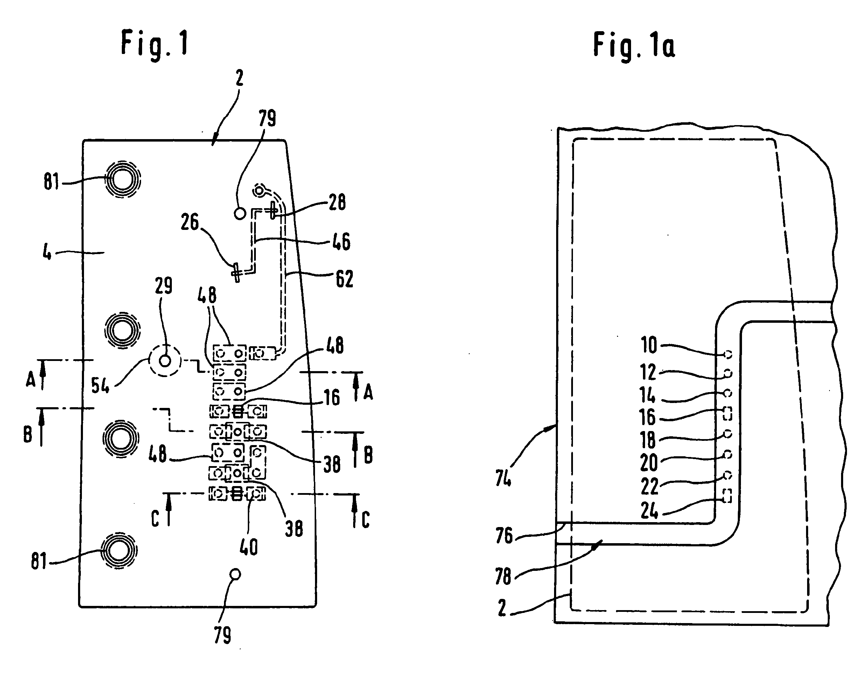

- Fig. 1: eine Draufsicht auf eine Ausführungsform der erfindungsgemäßen Sensorkarte,
- Fig. 1 a: eine Draufsicht auf eine Anordnung mit einer Sensorkarte und einer strukturierten Platte,
- Fig. 2: eine perspektivische Explosionsdarstellung der Sensorkarte von Fig. 1,
- Fig. 3: eine Schnittansicht entlang der Schnittlinie A-A von Fig. 1 in vergrößerter Darstellung,
- Fig. 4: eine Schnittansicht entlang der Schnittlinie B-B von Fig. 1 in vergrößerter Darstellung und
- Fig. 5: eine Schnittansicht entlang der Schnittlinie C-C von Fig. 1 in vergrößerter Darstellung.

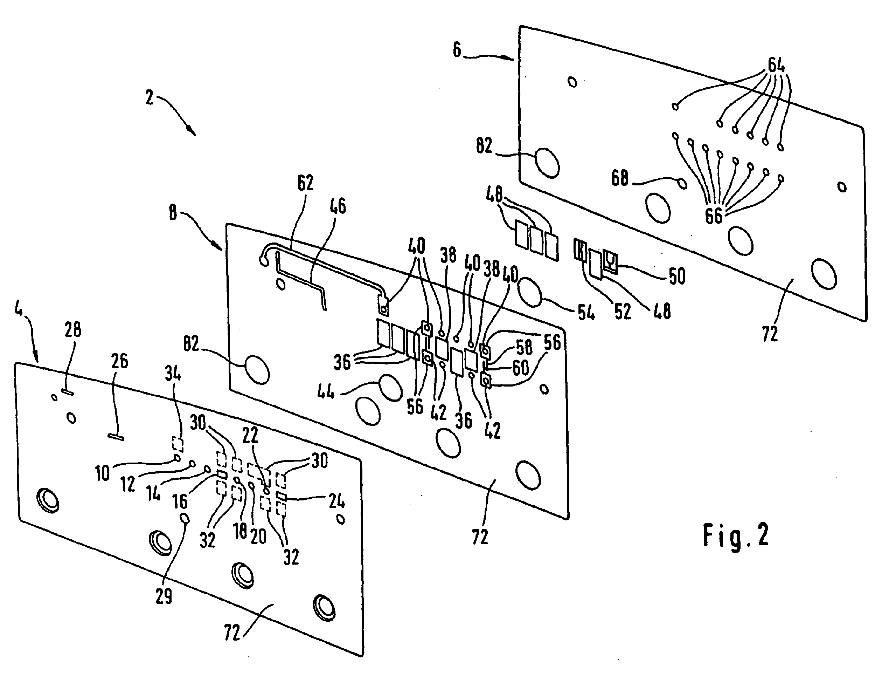

Fig. 1 zeigt eine Ausführungsform der erfindungsgemäßen Sensorkarte 2 in der Draufsicht, wobei der Aufbau der Sensorkarte 2 aus Gründen der Übersichtlichkeit an Hand von Fig. 2 näher erläutert werden soll.

Die Sensorkarte 2 weist eine Sensorfolie 4 und eine Deckfolie 6 auf, wobei die Sensorfolie 4 bei einer Messung der zu analysierenden Flüssigkeitsprobe (nicht dargestellt) zugewandt ist, wohingegen die Deckfolie 6 der Flüssigkeitsprobe abgewandt ist. Zwischen der Sensorfolie 4 und der Deckfolie 6 ist eine Zwischenfolie 8 angeordnet. Bei den Folien 4, 6, 8 handelt es sich in der vorliegenden Ausführungsform um Polyesterfolien, die eine Dicke zwischen 80 und 120 µm haben und flächig miteinander verbunden sind.

Die Sensorfolie 4 weist mehrere in einer Linie angeordnete Öffnungen 10 bis 24 auf. Des weiteren sind zwei voneinander beabstandete längliche Öffnungen 26, 28 sowie eine Belüftungsöffnung 29 in der Sensorfolie 4 vorgesehen.

Auf der der Zwischenfolie 8 zugewandten Seite der Sensorfolie 4 sind ferner aufgedruckte elektrische Leiterbahnen (gestrichelt dargestellt) vorgesehen, wobei jeweils eine Leiterbahn 30 oberhalb der Öffnungen 16 bis 24 und jeweils eine weitere Leiterbahn 32 unterhalb der Öffnungen 16, 18, 22 und 24 angeordnet ist. Darüber hinaus ist oberhalb der Öffnung 10 eine weitere Leiterbahn 34 für eine Referenzelektrode angeordnet.

Die Zwischenfolie 8 weist mehrere Aussparungen 36, 38, 40, 42 und 44 auf. Ferner ist eine längliche Aussparung 46 vorgesehen. In den Aussparungen 36, die bei der vorliegenden Ausführungsform rechteckig ausgebildet sind, sind ionenselektive Sensoren 48 angeordnet, während in die Aussparungen 38 Gassensoren, nämlich ein Sauerstoffsensor 50 und ein Kohlendioxidsensor 52, eingelegt sind. Innerhalb der kreisrunden Aussparung 44 ist eine Belüftungsmembran 54 vorgesehen.

Auf der Zwischenfolie 8 sind ferner zwei Leitfähigkeitssensoren 56 angeordnet, die jeweils zwei elektrisch leitfähige und voneinander beabstandete Leitungsabschnitte 58, 60 aufweisen, die auf der der Sensorfolie 4 zugewandten Seite aufgedruckt sind. Auf dieser Seite der Zwischenfolie 8 ist ferner ein langgestreckter Leitungsabschnitt 62 aufgedruckt, der als Referenzelektrode fungiert.

In der Deckfolie 6 sind wiederum mehrere Öffnungen 64, 66 zum elektrischen Abgriff sowie eine Belüftungsöffnung 68 vorgesehen.

Die Sensorkarte 2 ist mit einer Platte 74 verbunden (Fig. 1a), die mit weiteren Bauteilen innerhalb des Analysensystems bzw. des Meßinstrumentes eine austauschbare Einheit (Disposable) bildet. Zur exakten Positionierung der Sensorkarte 2 auf der Platte 74 sind in der Sensorkarte Positionierlöcher 79 vorgesehen, durch die Stifte greifen, die an der Platte vorgesehen sind.

Die Sensorkarte 2 ist derart mit der Platte 74 verbunden, dass die Sensorfolie 4 auf der Platte aufliegt. Die Platte 74 weist mindestens einen seitlich geöffneten Kanal 76 auf, der von einer Nut gebildet werden kann. Dadurch wird ein Strömungskanal 78 für die Flüssigkeitsprobe geschaffen, der von der Wandung des Kanals 76 der Platte 76 einerseits und der Innenseite der Sensorfolie 4 andererseits begrenzt ist. Der Strömungskanal 78 führt dabei entlang der in einer Linie angeordneten Öffnungen 10 bis 24 in der Sensorfolie 4, so dass die Flüssigkeits- oder Gasprobe mit den dahinterliegenden Sensoren 48, 50, 52, 56 in Kontakt treten kann.

Die beiden separaten und voneinander beabstandeten länglichen Öffnungen 26 und 28 in der Sensorfolie 4 stehen mit jeweils einem Abschnitt der länglichen Aussparung 46 in der Zwischenfolie 8 in Strömungsverbindung, so dass Flüssigkeiten oder Gase, beispielsweise Reagenzlösung als Innenelektrolyt der Referenzelektrode der ionenselektiven Sensoren oder ggf. auch Probenflüssigkeit von einer Öffnung 26 bzw. 28 durch die längliche Aussparung 46 zu der anderen Öffnung 28 bzw. 26 strömen kann. Vorteilhafterweise erstrecken sich die beiden länglichen Öffnungen 26, 28 jeweils quer, vorzugsweise im rechten Winkel zu der länglichen Aussparung 46 in der Zwischenfolie 8, wie dies in Fig. 1 dargestellt ist.

Darüber hinaus weist die Sensorkarte Bereiche 81 auf, an denen nicht dargestellte Betätigungsorgane zum Öffnen nicht dargestellter Flüssigkeitsbeutel angreifen, die in dem Disposable vorgesehen sind. In diesen Bereichen 81 ist die Sensorfolie 4 tiefgezogen, während in der Zwischenfolie 8 und der Deckfolie 6 Öffnungen 82 vorgesehen sind. Dadurch wird erreicht, dass die an diesen Bereichen der Sensorkarte angreifenden Betätigungorgane keinen Druck auf die Sensorkarte ausüben, solange sie nicht betätigt werden.

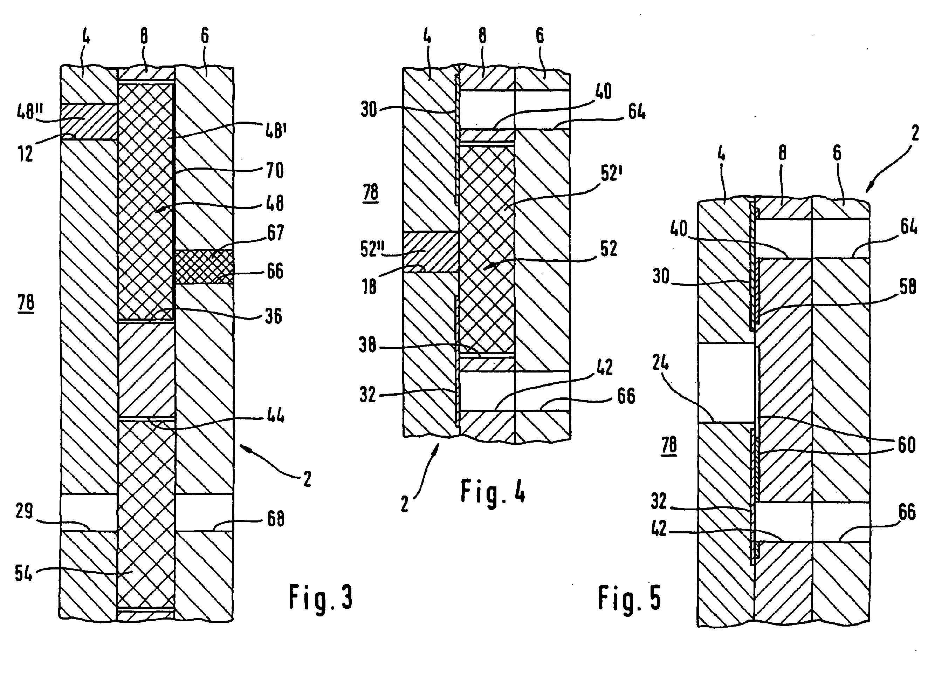

Die Funktionsweise sowie die Vorteile der vorliegenden Ausführungsform sollen nun an Hand der Fig. 3 bis 5 erläutert werden.

Fig. 3 zeigt den zwischen der Sensorfolie 4 und der Deckfolie 6 angeordneten ionenselektiven Sensor 48, der aus einem Trägermaterial 48' besteht, das im Bereich der Öffnung 12 mit einer ionenselektiven Reagenzmischung (Cocktail) 48" aktiviert oder funktionalisiert ist. Das ionenselektive Cocktail füllt dabei die Öffnung vollständig aus und dichtet den Sensor gegenüber der Sensorfolie ab.

Das Trägermaterial 48' des ionenselektiven Sensors 48 ist innerhalb der Aussparung 36 in der Zwischenfolie 8 angeordnet, so dass die Sensorkarte 2 im Bereich des ionenselektiven Sensors 48 nicht dicker als in anderen Bereichen ist, in denen kein Sensor vorgesehen ist. Zu diesem Zweck entspricht die Dicke der Zwischenfolie 8 der Dicke des Trägermaterials des Sensors. Die Sensorkarte hat somit den Vorteil, dass diese eine homogene Dicke aufweist. Eine derart eben ausgebildete Sensorkarte 2 kann besonders einfach und sicher flächig an der Platte mit Nuten befestigt werden, mit der die Sensorkarte 2 die Strömungskanäle ausbildet, so dass eine hohe Dichtigkeit der Strömungskanäle gewährleistet ist. Entsprechendes gilt auch für die später beschriebenen anderen Sensoren sowie für die Belüftungsmembran 54, die ebenfalls in Fig. 3 dargestellt ist.

Der ionenselektive Sensor 48 kann über die dem Sensor 48 zugeordnete Öffnung 66 in der Deckfolie 6 elektrisch abgegriffen werden, um entsprechende Signale an die nicht dargestellte Auswerteinheit des Analysensystems weiterzuleiten. Darüber hinaus weist der ionenselektive Sensor 48 an seiner der Öffnung 66 zum elektrischen Abgriff zugewandten Seite eine leitfähige Beschichtung 70 auf, die den elektrischen Abgriff vereinfacht. Auf der anderen Seite kann der ionenselektive Sensor 48 im Bereich der Öffnung 12 in der Sensorfolie 4 mit der Flüssigkeitsprobe (nicht dargestellt) benetzt werden.

Wie aus den Fig. 3 und 1 ersichtlich, sind die Öffnung 12 in der Sensorfolie 4 und die Öffnung 66 zum elektrischen Abgriff zueinander versetzt angeordnet. Die Öffnung 66 zum elektrischen Abgriff ist, wie auch die anderen Öffnungen 64, 66 in der Deckfolie, mit einer ausgehärteten, elektrisch leitenden Paste 67, nämlich Kohlepaste, gefüllt, was das Abgreifen erleichtert und sicherer macht. Aus Gründen der Übersichtlichkeit wurde die Paste 67 lediglich an Hand der Öffnung 66 in Fig. 3 beispielhaft angedeutet.

Unten in Fig. 3 ist die Belüftungsmembran 54 dargestellt, die in der Aussparung 44 in der Zwischenfolie 8 einliegt. Die Belüftungsmembran 54 grenzt sensorfolienseitig an die Belüftungsöffnung 29 und deckfolienseitig an die Belüftungsöffnung 68 an. Da die Belüftungsöffnung 68 der Belüftung und nicht dem elektrischen Abgriff dient, ist diese nicht mit Kohlepaste gefüllt. Die Belüftungsmembran 54 ist, wie auch alle Sensoren, größer als die ihr zugeordnete Öffnung 29 ausgebildet, so dass diese sicher in der Aussparung 44 in der Zwischenfolie 8 einliegt.

Fig. 4 zeigt das zwischen der Sensorfolie 4 und der Deckfolie 6 angeordnete Trägermaterial 52' des Kohlendioxidsensors 52. Das Trägermaterial 52' des Kohlendioxidsensors 52 ist dabei innerhalb der Aussparung 38 in der Zwischenfolie 8 angeordnet, so dass bezüglich der Dicke der gesamten Sensorkarte 2 ebenfalls die unter Bezugnahme auf Fig. 3 beschriebenen Vorteile eintreten. Der Kohlendioxidsensor 52 steht einerseits mit der ersten Leiterbahn 30 auf der Sensorfolie 4 und andererseits mit der zweiten Leiterbahn 32 auf der Sensorfolie 4 in Kontakt. Die Leiterbahn 30 kann über die Öffnung 64 in der Deckfolie 6 und die Aussparung 40 in der Zwischenfolie 8 elektrisch abgegriffen werden, während der elektrische Abgriff der Leiterbahn 32 über die Öffnung 66 in der Deckfolie 6 und die Aussparung 42 in der Zwischenfolie 8 erfolgen kann. Man spricht hier auch von einer Durchkontaktierung. Auch die Aussparungen 40 und 42 sind mit Kohlepaste gefüllt, die aus o.g. Gründen nicht dargestellt ist.

Das Trägermaterial 52' des Kohlendioxidsensors 52 weist eine der Sensorfolie 4 zugewandte Beschichtung auf, vorzugsweise eine Bedruckung aus Silber/Silberchlorid- und einem pH-sensitiven Material. Als Aktivierung oder Funktionalisierung ist auf der Beschichtung ein Innenelektrolyt 52" und die gaspermeable Membran 52" aufgebracht. Der Innenelektrolyt 52" und die gaspermeable Membran 52" füllen die Öffnung 18 in der Sensorfolie 4 aus.

Während der elektrische Abgriff von hinten, also über die Deckfolie, erfolgt, kann der Kohlendioxidsensor 52 an seiner der Sensorfolie 4 zugewandten Seite durch die Öffnung 18 in der Sensorfolie 4 mit der Probe in Kontakt kommen.

Fig. 5 zeigt den Leitfähigkeitssensor 56 (Fig. 2) im Querschnitt. Im Gegensatz zu den beiden unter Bezugnahme auf die Fig. 3 und 4 beschriebenen Sensoren ist der Leitfähigkeitssensor 56 nicht als Sensorinlay ausgebildet, sondern besteht vielmehr aus den eingangs beschriebenen Leitungsabschnitten 58 und 60, die auf der der Sensorfolie 4 zugewandten Seite der Zwischenfolie 8 aufgedruckt sind. Der eine Leitungsabschnitt 58 steht dabei mit einer ersten Leiterbahn 30, die sich auf der Sensorfolie 4 befindet, in Kontakt, während der andere Leitungsabschnitt 60 eine auf der Sensorfolie 4 befindliche zweite Leiterbahn 32 kontaktiert. Die erste Leiterbahn 30 kann über die Öffnung 64 in der Deckfolie 6 und die Aussparung 40 in der Zwischenfolie 8 elektrisch abgegriffen werden, während der elektrische Abgriff der zweiten Leiterbahn 32 über die Öffnung 66 in der Deckfolie 6 und die Aussparung 42 in der Zwischenfolie 8 erfolgen kann. Auch die Aussparungen 40 und 42 sind mit nicht dargestellter Kohlepaste gefüllt.

Beide Leitungsabschnitte 58, 60 erstrecken sich hinter die Öffnung 24 innerhalb der Sensorfolie 4 und können somit über diese Öffnung 24 mit der Flüssigkeitsprobe benetzt werden. Durch die in die Öffnung 24 eintretende Flüssigkeit werden die beiden Leitungsabschnitte 58, 60 elektrisch leitend verbunden, so dass die Leitfähigkeit der Flüssigkeitsprobe ermittelt werden kann.

Im Folgenden soll das erfindungsgemäße Verfahren an Hand der Fig. 2 erläutert werden. Zunächst werden die in Fig. 2 dargestellten Folien, nämlich die Sensorfolie 4, die Deckfolie 6 und die Zwischenfolie 8 in der dargestellten Weise zur Verfügung gestellt und angeordnet. Danach werden die fertigen Sensoren oder die später noch zu aktivierenden bzw. funktionalisierenden Teile der Sensoren 48, 50, 52, die als Sensorinlays ausgebildet sind, in den entsprechenden Aussparungen 36, 38 in der Zwischenfolie 8 angeordnet. Anschließend werden die übereinander angeordneten Folien 4, 6, 8 flächig miteinander verbunden, wobei das Verbinden durch Kleben, Heißlaminieren, Hitzeverschweißen oder Hochfrequenzschweißen erfolgen kann. Besonders einfach gestaltet sich das Einlegen der Sensoren, wenn die Folien 4, 6, 8 vor dem Einlegen der Sensorinlays bereits teilweise miteinander verbunden werden, indem beispielsweise der untere Rand 72 (Fig. 2) der Folien 4, 6, 8 anlaminiert wird.

Werden nur die Trägermaterialien von noch nicht fertigen Sensoren in die Aussparungen eingelegt, so werden die Sensoren erst nach dem Verbinden der Folien aktiviert oder funktionalisiert. Hierzu werden die verschiedenen analytspezifischen Reagenzlösungen zur Aktivierung oder Funktionalisierung der Sensoren durch die Öffnungen in der Sensorfolie auf die Trägermaterialien der jeweiligen Sensoren aufgebracht und getrocknet.

## Patentansprüche

1. Sensorkarte zur Bestimmung von Analyten in Flüssigkeits- oder Gasproben mit flächig miteinander verbundenen Folien aufweisend
eine der Probe zugewandte Sensorfolie (4) mit Öffnungen (10-24, 26,28,29),
eine der Probe abgewandte Deckfolie (6) und
mindestens einen Sensor oder einen Teil eines Sensors (48, 50, 52), der zwischen der Sensorfolie (4) und der Deckfolie (6) angeordnet ist,
wobei zwischen der Sensorfolie (4) und der Deckfolie (6) mindestens eine Zwischenfolie (8) mit Aussparungen (36, 3 8, 40, 42, 44,46) vorgesehen und der Sensor (48, 50, 52) in einer der Aussparungen (36, 38) angeordnet ist,
**dadurch gekennzeichnet, dass** die der Probe abgewandte Deckfolie (6) mit Öffnungen (64,66) zum elektrischen Abgriff des mindestens einen Sensors (48, 50, 52) versehen ist.

2. Sensorkarte nach Anspruch 1, **dadurch gekennzeichnet, dass** die Dicke der Zwischenteile (8) größer oder gleich der Dicke des Sensors oder des Teils des Sensors (48, 50, 52) ist.

3. Sensorkarte nach Anspruch 1 oder 2, **dadurch gekennzeichnet, dass** die Öffnungen (64, 66) zum elektrischen Abgriff mit einer ausgehärteten, elektrisch leitenden Paste (67) gefüllt sind.

4. Sensorkarte nach einem der vorangehenden Ansprüche, **dadurch gekennzeichnet, dass** der Sensor ein ionenselektiver Sensor (48) ist, der einerseits über eine der Öffnungen (66) in der Deckfolie (6) elektrisch abgreifbar und andererseits über eine der Öffnungen (10, 12, 14, 20) in der Sensorfolie (4) mit der Flüssigkeits- oder Gasprobe in Kontakt treten kann.

5. Sensorkarte nach Anspruch 4, **dadurch gekennzeichnet, dass** die Öffnung (66) zum elektrischen Abgriff in der Deckfolie (6) versetzt zu der Öffnung (10, 12, 14, 20) in der Sensorfolie (4) angeordnet ist.

6. Sensorkarte nach einem der Ansprüche 4 oder 5, **dadurch gekennzeichnet, dass** der ionenselektive Sensor (48) ein in der Aussparung (36) einliegendes Trägermaterial (48') aufweist, das im Bereich der Öffnung (10,12,14, 20) in der Sensorfolie (4) mit einer ionenselektiven Reagenzmischung (48") aktiviert ist.

7. Sensorkarte nach Anspruch 6, **dadurch gekennzeichnet, dass** das Trägermaterial an seiner der Öffnung (66) zum elektrischen Abgriff zugewandten Seite eine leitfähige Beschichtung (70) aufweist.

8. Sensorkarte nach einem der vorangehenden Ansprüche, **dadurch gekennzeichnet, dass** die Sensorfolie (4) der Zwischenfolie (8) zugewandte, aufgedruckte elektrische Leiterbahnen (30, 32, 34) aufweist, wobei die Leiterbahnen (30, 32, 34) jeweils über die Öffnungen (64, 66) zum elektrischen Abgriff und mindestens eine der Aussparungen (40, 42) in der Zwischenfolie (8) elektrisch abgreifbar sind.

9. Sensorkarte nach Anspruch 8, **dadurch gekennzeichnet, dass** der Sensor ein Gassensor ist, der mit einer ersten Leiterbahn (30) und einer zweiten Leiterbahn, (32) auf der Sensorfolie (4) in Kontakt steht und über eine der Öffnungen (18, 22) in der Sensorfolie (4) mit der Flüssigkeits- oder Gasprobe in Kontakt treten kann.

10. Sensorkarte nach Anspruch 9, **dadurch gekennzeichnet, dass** der Gassensor ein Kohlendioxidsensor (52) ist, insbesondere ein Kohlendioxidsensor, der ein Trägermaterial (52') aufweist, das eine der Sensorfolie (4) zugewandte Beschichtung, vorzugsweise eine Bedruckung aus Silber/Silberchlorid- und einem pH-sensitiven Material, aufweist, wobei auf der Beschichtung ein Innenelektrolyt (52") und eine gaspermeable Membran (52") als Aktivierung aufgebracht ist.

11. Sensorkarte nach Anspruch 9, **dadurch gekennzeichnet, dass** der Gassensor ein Sauerstoffsensor (50) ist, insbesondere ein Sauerstoffsensor, der ein Trägermaterial mit hierauf aufgebrachten, vorzugsweise aufgedruckten Schichten eines als Arbeitselektrode und eines als Referenzelektrode geeigneten Materials aufweist, wobei auf den Schichten ein Innenelektrolyt und eine gaspermeable Membran als Aktivierung aufgebracht sind.

12. Sensorkarte nach Anspruch 8, **dadurch gekennzeichnet, dass** der Sensor ein Leitfähigkeitssensor (56) ist, der zwei elektrisch leitfähige, voneinander beabstandete Leitungsabschnitte (58, 60) aufweiset, die auf der der Sensorfolie (4) zugewandten Seite der Zwischenfolie (8) aufgedruckt sind, wobei der eine Leitungsabschnitt (58) mit einer ersten Leiterbahn (30) auf der Sensorfolie (4) und der andere Leitungsabschnitt (60) mit einer zweiten Leiterbahn (32) auf der Sensorfolie (4) in Kontakt steht und die beiden Leitungsabschnitte (58, 60) über eine Öffnung (16, 24) innerhalb der Sensorfolie (4) durch die Flüssigkeitsprobe benetzbar und elektrisch leitend verbindbar sind.

13. Sensorkarte nach einem der vorangehenden Ansprüche, **dadurch gekennzeichnet, dass** in mindestens einer der Aussparungen (44) in der Zwischenfolie (8) eine Belüftungsmealbran (54) angeordnet ist, wobei die Belüftungsmembran (54) einerseits an eine Belüftungsöffnung (29) in der Sensorfolie (4) und andererseits an eine Belüftungsöffnung (68) in der Deckfolie (6) angrenzt.

14. Sensorkarte nach einem der vorangehenden Ansprüche, **dadurch gekennzeichnet, dass** eine der Aussparungen (46) in der Zwischenfolie (8) länglich ausgebildet ist, wobei die längliche Aussparung (46) mit mindestens zwei separaten Öffnungen (26, 28) in der Sensorfolie (4) in Strömungsverbindung steht.

15. Sensorkarte nach Anspruch 14, **dadurch gekennzeichnet, dass** die beiden separaten Öffnungen (26, 28) länglich ausgebildet sind und quer, vorzugsweise im rechten Winkel, zu der länglichen Aussparung (46) in der Zwischenfolie (8) verlaufen.

16. Anordnung zur Bestimmung von Analyten in Flüssigkeits- oder Gasproben aufweisend
eine Sensorkarte nach einem der vorangehenden Ansprüche und
eine Platte mit mindestens einem seitlich geöffneten Kanal,
wobei die Sensorfolie der Sensorkarte unter Ausbildung eines seitlich geschlossenen Strömungskanals für die Probe an der Platte befestigt ist.

17. Verfahren zur Herstellung einer Sensorkarte zur Bestimmung von Analyten in Flüssigkeitsproben mit flächig miteinander verbundenen Folien mit den Verfahrensschritten
ZurverFügtxngstellen einer Sensorfolie mit Öffnungen, einer Deckfolie mit Öffnungen zum elektrischen Abgriff und mindestens einer Zwischenfolie, in der Aussparungen vorgesehen sind,
Anordnen mindestens eines Sensors oder eines Teils eines Sensors in einer der Aussparungen in der Zrwischenfolie und
flächiges Verbinden der übereinander angeordneten Folien, wobei die Zwischenfolie zwischen Sensorfolie und Deckfolie angeordnet wird.

18. Verfahren nach Anspruch 17, **dadurch gekennzeichnet, dass** zumindest eine der Folien vor dem Anordnen des Sensors oder des Teils des Sensors in einer der Aussparungen mit der Zwischenfolie teilweise flächig verbunden wird.

19. Verfahren nach Anspruch 18, **dadurch gekennzeichnet, dass** sowohl die Sensorfolie als auch die Deckfolie vor dem Anordnen des Sensors oder des Teils des Sensors in einer der Aussparungen mit der Zwischenfolie teilweise flächig verbunden werden.

20. Verfahren nach einem der Ansprüche 17 bis 19, **dadurch gekennzeichnet, dass** vor dem Zurverfi.igungstellen der Sensorfolie Leiterbahnen auf die der Zwischenfolie zuzuwendende Seite der Sensorfolie aufgedruckt werden, vorzugsweise durch Siebdruck.

21. Verfahren nach einem der Ansprüche 17 bis 20, **dadurch gekennzeichnet, dass** vor dem Zurverfügungstellen der Zwischenfolie Leitungsabschnitte auf die der Sensorfolie zuzuwendende Seite der Zwischenfolie aufgedruckt werden, vorzugsweise durch Siebdruck.

22. Verfahren nach einem der Ansprüche 17 bis 21, **dadurch gekennzeichnet, dass** das Verbinden durch Kleben, Heißlaminieren, Hitzeversehweißen oder Hochfrequenzschweißen erfolgt.

## Claims

1. Sensor card for determining analytes in liquid or gaseous samples, in which films are connected together over their area, having a sensor film (4), adjacent the sample, which has openings (10-14, 26, 28, 29), having a covering film (6) remote from the sample, and having at least one sensor or one part of a sensor (48, 50, 52) which is arranged between the sensor film (4) and the covering film (6), at least one intermediate film (8) which has apertures (36, 38, 40, 42, 44, 46) being provided between the sensor film (4) and the covering film (6) and the sensor (48, 50, 52) being arranged in one of the apertures (36, 38), **characterised in that** the covering film (6) remote from the sample is provided with openings (64, 66) for electrical pick-off from the at least one sensor (48, 50, 52).

2. Sensor card according to claim 1, **characterised in that** the thickness of the intermediate film (8) is equal to or greater than the thickness of the sensor or the part of the sensor (48, 50, 52),

3. Sensor card according to claim 1 or 2, **characterised in that** the openings (64, 66) for electrical pick-off are filled with a cured electrically conductive paste (67).

4. Sensor card according to one of the preceding claims, **characterised in that** the sensor is an ion-selective sensor (48) from which on the one hand electrical pick-off is possible through one of the openings (66) in the covering film (6) and which on the other hand is able to make contact with the liquid or gaseous sample through one of the openings (10, 12, 14, 20) in the sensor film (4).

5. Sensor card according to claim 4, **characterised in that** the opening (66) for electrical pick-off in the covering film (6) is arranged in an offset position from the opening (10, 12, 14, 20) in the sensor film (4).

6. Sensor card according to either of claims 4 and 5, **characterised in that** the ion-selective sensor (48) has a substrate material (48') which is situated in the aperture (36) and which is activated by an ion-selective reagent mixture (48") in the region of the opening (10, 12, 14, 20) in the sensor film (4).

7. Sensor card according to claim 6, **characterised in that** the substrate material has a conductive coating (70) on its side adjacent the opening (66) for electrical pick-off.

8. Sensor card according to one of the preceding claims, **characterised in that** the sensor film (4) has printed electrical conductors (30, 32, 34) adjacent the intermediate film (8), electrical pick-off from the conductors (30, 32, 34) being possible through each of the openings (64, 66) for electrical pick-off and through at least one of the apertures (40, 42) in the intermediate film (8).

9. Sensor card according to claim 8, **characterised in that** the sensor is a gas sensor which is in contact with a first conductor (30) and a second conductor (32) on the sensor film (4) and which is able to come into contact with the liquid or gaseous sample through one of the openings (18, 22) in the sensor film (4).

10. Sensor card according to claim 9, **characterised in that** the gas sensor is a carbon dioxide sensor (52), and in particular a carbon dioxide sensor which has a substrate material (52') which has a coating adjacent the sensor film (4) and preferably a printed coating of silver/silver chloride material and of a pH-sensitive material, a reference electrolyte (52") and a gas-permeable membrane (52") being applied to the coating as activating means.

11. Sensor card according to claim 9, **characterised in that** the gas sensor is an oxygen sensor (50) and in particular an oxygen sensor which has a substrate material which has applied to it, and preferably printed on it, layers of a material which is suitable to act as a working electrode and of a material which is suitable to act as a reference electrode, a reference electrolyte and a gas-permeable membrane being applied to the coating as activating means.

12. Sensor card according to claim 8, **characterised in that** the sensor is a conductivity sensor (56) which has two electrically conductive portions of electrical line (58, 60) which are spaced apart from one another and which are printed on the side of the intermediate film (8) adjacent the sensor film (4), one portion of electrical line (58) being in contact with a first conductor (30) on the sensor film (4) and the other portion of electrical line (60) being in contact with a second conductor (32) on the sensor film (4) and the two portions of electrical line (58, 60) being able to be wetted, and connected by an electrically conductive connection, by the liquid sample through an opening (16, 24) within the sensor film (4).

13. Sensor card according to one of the preceding claims, **characterised in that** an air-admission membrane (54) is arranged in at least one of the apertures (44) in the intermediate film (8), the air-admission membrane (51) adjoining on the one hand an air-admission opening (29) in the sensor film (4) and on the other hand an air-admission opening (68) in the covering film (6).

14. Sensor card according to one of the preceding claims, **characterised in that** one (46) of the apertures in the intermediate film (8) is of an elongated form, the elongated aperture (46) being in flow-permitting connection with at least two separate openings (26, 28) in the sensor film (4).

15. Sensor card according to claim 14, **characterised in that** the two separate openings (26, 28) are of an elongated form and extend transversely, and preferably at right angles, to the elongated aperture (46) in the intermediate film (8).

16. Arrangement for determining analytes in liquid or gaseous samples, having a sensor card according to one of the preceding claims and a plate having at least one passage which is open laterally, wherein the sensor film of the sensor card is fastened to the plate to form a passage for the flow of the sample which is closed off laterally.

17. Method for producing a sensor card for determining analytes in liquid samples in which films are connected together over their area, the method having the following method steps
supply of a sensor film having openings, of a covering film having openings for electrical pick-off, and of at least one intermediate film in which apertures are provided,
arranging of at least one sensor or one part of a sensor in one of the apertures in the intermediate film, and
connection over their area of the films when arranged one above the other, the intermediate film being arranged between the sensor film and the covering film.

18. Method according to claim 17, **characterised in that**, before the sensor or the part of a sensor is arranged in one of the apertures, at least one of the films is connected to the intermediate film over part of its area.

19. Method according to claim 18, **characterised in that**, before the sensor or the part of a sensor is arranged in one of the apertures, both the sensor film and the covering film are connected to the intermediate film over part of their area.

20. Method according to one of claims 17 to 19, **characterised in that**, before the sensor film is supplied, conductors are printed on that side of the sensor film which is to be adjacent the intermediate film, preferably by screen printing.

21. Method according to one of claims 17 to 20, **characterised in that**, before the intermediate film is supplied, portions of electrical line are printed on that side of the intermediate film which is to be adjacent the sensor film, preferably by screen printing.

22. Method according to one of claims 17 to 21, **characterised in that** the connection is made by adhesive bonding, hot lamination, heat welding or high-frequency welding.

## Revendications

1. Carte sensorielle destinée à déterminer les substances à analyser dans des échantillons de liquide ou de gaz, munie de feuilles reliées entre elles de manière plane, comportant
✔ une feuille sensorielle (4), orientée vers l'échantillon et munie d'orifices (10-24, 26, 28, 29),
✔ une feuille de recouvrement (6) détournée de l'échantillon, et
✔ au moins un capteur ou une partie d'un capteur (48, 50, 52), qui est disposé entre la feuille sen-sorielle (4) et la feuille de recouvrement (6),
✔ au moins une feuille intermédiaire (8) avec des évidements (36, 38, 40, 42, 44, 46) étant pré-vue entre la feuille sensorielle (4) et la feuille de recouvrement (6), et le capteur (48, 50, 52) étant logé dans l'un des évidements (36, 38),
**caractérisée en ce que** la feuille de recouvrement (6), détournée de l'échantillon, est munie d'ori-fices (64, 66) pour le branchement électrique dudit au moins un capteur (48, 50, 52).

2. Carte sensorielle selon la revendication 1, **caractérisée en ce que** l'épaisseur de la feuille intermédiaire (8) est supérieure ou égale à l'épaisseur du capteur ou de la partie du capteur (48, 50, 52).

3. Carte sensorielle selon la revendication 1 ou 2, **caractérisée en ce que** les orifices (64, 66) pour le branchement électrique sont remplis d'une pâte (67) électroconductrice durcie.

4. Carte sensorielle selon l'une quelconque des revendications précédentes, **caractérisée en ce que** le capteur est un capteur ionosensible (48) qui, d'une part, peut être branché électriquement via un des orifices (66) dans la feuille de recouvrement (6) et, d'autre part, peut entrer en contact avec l'échantillon de liquide ou de gaz via les orifices (10, 12, 14, 20) dans la feuille sensorielle (4).

5. Carte sensorielle selon la revendication 4, **caractérisée en ce que** l'orifice (66) pour le branchement électrique dans la feuille de recouvrement (6) est décalé par rapport à l'orifice (10, 12, 14, 20) dans la feuille sensorielle (4).

6. Carte sensorielle selon la revendication 4 ou 5, **caractérisée en ce que** le capteur ionosensible (48) comporte un matériau de base (48') inséré dans l'évidement (36), lequel est activé avec un mélange réactif (48") ionosensible dans la zone de l'orifice (10, 12, 14, 20) dans la feuille sensorielle (4).

7. Carte sensorielle selon la revendication 6, **caractérisée en ce que** le matériau de base com-porte un revêtement conducteur (70) sur sa face orientée vers l'orifice (66) pour le branchement électrique.

8. Carte sensorielle selon l'une quelconque des revendication précédentes, **caractérisée en ce que** la feuille sensorielle (4) comporte des pistes conductrices (30, 32, 34) électriques imprimées, orientées vers la feuille intermédiaire (8), lesdites pistes conductrices (30, 32, 34) pouvant être branchées électriquement via les orifices (64, 66) pour le branchement électrique et au moins un des évidements (40, 42) dans la feuille intermédiaire (8).

9. Carte sensorielle selon la revendication 8, **caractérisé en ce que** le capteur est un capteur de gaz, qui est en contact avec une première piste conductrice (30) et une deuxième piste con-ductrice (32) sur la feuille sensorielle (4) et qui peut entrer en contact avec l'échantillon de liquide ou de gaz via l'un des orifices (18, 22) dans la feuille sensorielle (4).

10. Carte sensorielle selon la revendication 9, **caractérisée en ce que** le capteur de gaz est un capteur de dioxyde de carbone (52), en particu-lier un capteur de dioxyde de carbone qui est muni d'un matériau de base (52'), qui comporte un revêtement orienté vers la feuille sensorielle (4), de préférence une impression avec un maté-riau à base d'argent ou de chlorure d'argent et un matériau sensible au pH, un électrolyte interne (52") et une membrane (52") perméable au gaz étant déposés sur le revêtement pour réaliser une activation.

11. Carte sensorielle selon la revendication 9, **caractérisée en ce que** le capteur de gaz est un capteur d'oxygène (50), en particulier un capteur d'oxygène qui est muni d'un matériau de base, sur lequel sont déposées, de préférence imprimées, des couches d'un matériau approprié à former une électrode de travail et d'un matériau approprié à former une électrode de référence, un électrolyte interne et une membrane perméable au gaz étant déposés sur les couches pour réaliser une acti-vation.

12. Carte sensorielle selon la revendication 8, **caractérisée en ce que** le capteur est un capteur conducteur (56), qui comporte deux tronçons d'interconnexion (58, 60) électroconducteurs, qui sont situés à distance l'un de l'autre et qui sont imprimés sur la face de la feuille inter-médiaire (8), orientée
vers la feuille senso-rielle (4), l'un des tronçons d'interconnexion (58) étant en contact avec une première piste conductrice (30) sur la feuille sensorielle (4), et l'autre tronçon d'interconnexion (60) étant en contact avec une deuxième piste conductrice (32) sur la feuille sensorielle (4), et les deux tronçons d'interconnexion (58, 60) étant aptes à être mouillés par l'échantillon de liquide et étant aptes à être reliés entre eux de manière électroconductrice via un orifice (16, 24) à l'intérieur de la feuille sensorielle (4).

13. Carte sensorielle selon l'une quelconque des revendications précédentes, **caractérisée en ce qu'**une membrane d'aération (54) est disposée dans au moins un des évidements (44) dans la feuille intermédiaire (8), ladite membrane d'aération (54) étant adjacente, d'une part, à un orifice d'aération (29) dans la feuille sensorielle (4) et, d'autre part, à un orifice d'aération (68) dans la feuille de recouvrement (6).

14. Carte sensorielle selon l'une quelconque des revendications précédentes, **caractérisée en ce que** l'un des évidements (46) dans la feuille intermédiaire (8) a une forme allongée, ledit évidement (46) allongé étant en liaison fluidique avec au moins deux orifices (26, 28) séparés dans la feuille sensorielle (4).

15. Carte sensorielle selon la revendication 14, **caractérisée en ce que** les deux orifices (26, 28) séparés ont une forme allongée et s'étendent transversalement, de préférence à angle droit, par rapport à l'évidement (46) allongé dans la feuille intermédiaire (8).

16. Agencement destiné à déterminer les subs-tances à analyser dans des échantillons de liquide ou de gaz, comportant :
✔ une carte sensorielle selon l'une des revendications précédentes,
✔ une plaquette avec au moins un conduit ouvert latéralement,
dans lequel la feuille sensorielle de la carte sensorielle est fixée sur la plaquette moyennant la formation d'un conduit d'écoulement fermé latéralement pour l'échantillon.

17. Procédé pour la réalisation d'une carte sensorielle destinée à déterminer les substances à analyser dans des échantillons de liquide, munie de feuilles reliées entre elles de manière plane, comportant les étapes :
✔ mise à disposition d'une feuille sensorielle avec des orifices, d'une feuille de recouvre-ment avec des orifices pour le branchement électrique et au moins d'une feuille intermé-diaire, dans laquelle sont prévus des évide-ments,
✔ mise en place d'au moins un capteur ou d'une partie d'un capteur dans l'un des évidements dans la feuille intermédiaire, et
✔ assemblage plan des feuilles superposées, la feuille intermédiaire étant disposée entre la feuille sensorielle et la feuille de recouvre-ment.

18. Procédé selon la revendication 17, caracté-risé en ce qu'au moins une des feuilles est assemblée en partie de manière plane avec la feuille intermédiaire avant la mise en place du capteur ou de la partie du capteur dans l'un des évidements.

19. procédé selon la revendication 18, caracté-risé en ce que la feuille sensorielle, de même que la feuille de recouvrement sont assemblées en partie de manière plane avec la feuille intermédiaire avant la mise en place du capteur de la partie du capteur dans l'un des évidements.

20. Procédé selon l'une quelconque des revendica-tions 17 à 19, **caractérise en ce qu'**avant la mise à disposition de la feuille sensorielle, des pistes conductrices sont imprimées, de préférence par sérigraphie, sur la face de la feuille sensorielle orientée vers la feuille intermé-diaire.

21. Procédé selon l'une quelconque des revendica-tions 17 à 20, **caractérisé en ce qu'**avant la mise à disposition de la feuille intermédiaire, des tronçons d'interconnexion sont imprimés, de préférence par sérigraphie, sur la face de la feuille intermédiaire, orientée vers la feuille sensorielle.

22. Procédé selon l'une quelconque des revendica-tions 17 à 22, **caractérisé en ce que** l'assemblage est effectué par collage, laminage à chaud, soudage à chaud ou soudage à haute fréquence.
